(19) Europäisches Patentamt
European Patent Office
Office européen des brevets

(11) Numéro de publication : **0 342 084 B1**

# (12) FASCICULE DE BREVET EUROPEEN

(45) Date de publication du fascicule du brevet : **22.07.92 Bulletin 92/30**

(51) Int. Cl.$^5$ : **C07C 69/14,** C07C 67/36

(21) Numéro de dépôt : **89401160.0**

(22) Date de dépôt : **24.04.89**

## (54) Procédé de préparation d'acétate de méthyle à partir de formiate de méthyle.

(30) Priorité : **25.04.88 FR 8805462**

(43) Date de publication de la demande : **15.11.89 Bulletin 89/46**

(45) Mention de la délivrance du brevet : **22.07.92 Bulletin 92/30**

(84) Etats contractants désignés : **AT BE CH DE ES FR GB GR IT LI LU NL SE**

(56) Documents cités :
**DE-A- 1 003 710**
**FR-A- 2 079 035**

(73) Titulaire : **SOLLAC**
**Immeuble Elysées-La Défense 29, le Parvis**
**F-92072 Puteaux (FR)**

(72) Inventeur : **Cordier Jean-Armand**
**La Rougeville, 48 rue Henri Barbusse**
**F-59880 St Saulve (FR)**
Inventeur : **Petit,Francis Paul**
**13 Allée de la Clairière**
**F-59650 Villeneuve d'Asq (FR)**
Inventeur : **Castenet,Yves**
**29 rue de la Comédie**
**F-59650 Villeneuve d'Asq (FR)**
Inventeur : **Melloul,Serge**
**138 Boulevard Gambetta**
**F-75010 Paris (FR)**
Inventeur : **Mortreux,André**
**17 rue Gambetta**
**F-59650 Hem (FR)**

(74) Mandataire : **Le Guen, Gérard et al**
**CABINET LAVOIX 2, place d'Estienne d'Orves**
**F-75441 Paris Cédex 09 (FR)**

EP 0 342 084 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation sélective d'acétate de méthyle à partir de formiate de méthyle.

L'isomérisation du formiate de méthyle en acide acétique selon le schéma suivant est une réaction bien connue.

$$HCOOCH_3 \xrightarrow[CO]{\text{catalyseur}} CH_3COOH$$

Elle est catalysée, en phase liquide par de nombreux métaux de transition du groupe VIII, parmi ceux-ci l'iridium, le rhodium, le cobalt, le ruthénium et le palladium sont le plus souvent cités dans la littérature. La réaction s'effectue généralement en présence d'un promoteur iodé ($CH_3I$,HI), à une température variant entre 150 et 200°C et le plus souvent sous pression de monoxyde de carbone dont le rôle consiste uniquement à stabiliser l'espèce catalytique.

La sélectivité en acide acétique qui peut être bonne (99%), dépend des conditions expérimentales : catalyseurs, solvant, promoteur, cocatalyseur ...

L'influence de ces divers paramètres a été étudié par M. Röper et coll. (Erdol und Kohle, Erdgas Petrochem, 38 (1), 1985), le principal sous produit étant toujours l'acétate de méthyle qui se forme en proportion notable essentiellement lorsque l'activité du système catalytique est faible (vitesse du cycle inférieure à 20 $h^{-1}$).

La présente invention vise à fournir un procédé de synthèse de l'acétate de méthyle ayant une bonne sélectivité en partant du formiate de méthyle. Les réactions qui peuvent être mises en jeu sont les suivantes :

$$2HCOOCH_3 \xrightarrow{\text{catalyseur}} CH_3COOCH_3 + HCOOH$$
$$HCOOH \longrightarrow H_2 + CO_2$$
$$HCOOH \longrightarrow H_2O + CO$$

Ainsi dans les conditions de la réaction, l'acide formique initialement coproduit se décompose pour donner du CO, de l'hydrogène, du $CO_2$ et de l'eau.

L'invention est caractérisée en ce que l'on fait réagir le formiate de méthyle à une température de 170 à 220°C en présence de :

a) un catalyseur à base de rhodium,

b) un promoteur iodé choisi parmi les iodures alcalins, alcalino-terreux, de phosphonium ou d'ammonium et les composés covalents d'iode additionnés d'une phosphine ou d'une amine tertiaire, ce promoteur étant présent à une concentration molaire de 0,05 à 1 mole/litre,

c) un solvant choisi parmi un N-alkyl amide cyclique.

Comme exemple de catalyseur à base de rhodium on peut citer $RhCl_3$, $3H_2O$ - $RhCl_2(CO)_4$ ou Rh (CO)Cl($(PPh_3)_2$).

Le promoteur iodé est de préférence un iodure alcalin ou alcalino-terreux tel que LiI, NaI, KI, $CaI_2$...

Il peut être éventuellement covalent : $I_2$, $CH_3I$, HI... Dans ce cas, l'addition de phosphine de formule $PR_3$, ou d'amine tertiaire $NR_3$ (R étant un groupe alkyle ou aryle, notamment $PPh_3$) dans un rapport de 1 à 2 avec le promoteur, se révèle bénéfique.

Le solvant est utilisé avantageusement en une proportion molaire par rapport au formiate représentant de 10 à 50%. Comme exemple de N-(alkyl en $C_1$-$C_6$) amide cyclique on peut citer la N-méthylpyrrolidone, la N-éthylpyrrolidone, et la N,N-diméthylimidazolidinone.

La réaction peut s'effectuer sans pression initiale de CO. Il est cependant avantageux de travailler sous une pression de CO comprise entre $1.10^5$ et $20.10^5$ Pa, notamment pour éviter la décomposition du formiate en CO et méthanol et améliorer la sélectivité en acétate de méthyle. Des pressions supérieures sont également possibles mais se traduisent le plus souvent par une baisse d'activité.

Le catalyseur est avantageusement utilisé en une proportion molaire par rapport au formiate de méthyle comprise entre $10^{-3}$ et $4.10^{-4}$.

Les exemples suivants illustrent le procédé selon l'invention :

Exemple 1 :

Dans un autoclave de 100 $cm^3$ sont introduits 1g de LiI en solution dans 30 $cm^3$ de formiate de méthyle et 10 $cm^3$ de N-méthylpyrrolidone (NMP). L'autoclave est purgé par un courant de CO puis pressurisé à $5.10^5$ Pa. Puis il est chauffé à 180°C sous agitation ; lorsque sa température s'est stabilisée on injecte rapidement (2 minutes) avec une pompe doseuse 0,06 g de $RhCl_3$, $3H_2O$ dissous dans 10 $cm^3$ de NMP. La transformation de formiate de méthyle est suivie par analyse chromatographique en phase gazeuse de prélèvements périodiques, la fin de l'injection du catalyseur étant pris comme instant initial. Au bout de 4 heures de réaction l'agitation est arrêtée, l'autoclave est refroidi, les phases liquides et gazeuses sont analysées par chromatographie.

Résultats :

| liquide (% molaire) | | gazeuse (% molaire) | |
|---|---|---|---|
| MeOMe | = 1,2 | $H_2$ | = 2 |
| MeOH | = 1,7 | CO | = 48 |
| $HCOOCH_3$ | = 78 | $CO_2$ | = 34 |
| $CH_3COOCH_3$ | = 18,3 | $CH_4$ | = 16 |

On a reporté dans le tableau I ci-après les résultats cinétiques obtenus dans l'exemple 1, ainsi que d'autres résultats obtenus en utilisant un protocole expérimental similaire à celui décrit dans l'exemple 1.

TABLEAU 1

| Ex. N° | Conditions Expérimentales | t (h) | Conversion totale % | Sélectivités molaires % en acétate de méthyle | Vitesse de cycle ($h^{-1}$) |
|---|---|---|---|---|---|
| 1 | $RhCl_3,3H_2O$= | | | | |
| | 0,25 mmole | 0,1 | 8,7 | 53 | |
| | LiI=1g | 0,25 | 21 | 94,2 | 1680 |
| | FOMe=500 mmoles | 1 | 24,8 | 94,4 | 496 |
| | NMP=20cm$^3$ | 3 | 28,4 | 91,5 | 189 |
| | T=180°C | | | | |
| | PCO=1 MPa | | | | |
| 2 | $RhCl_3,3H_2O$= | | | | |
| | 0,25 mmole | 1,25 | 4,8 | 39,6 | |
| | $ICH_3$=1g | 2,5 | 11,3 | 83,2 | 90 |
| | $PPh_3$=1g | 4,5 | 16,8 | 73,7 | 75 |
| | FOMe=500 mmoles | | | | |
| | NMP=20cm$^3$ | | | | |
| | T=180°C | | | | |
| | PCO=1 MPa | | | | |
| 3 | $RhCl_3,3H_2O$= | | | | |
| | 0,25 mmole | 0,5 | 17,9 | 75 | 716 |
| | LiI=0,15g | 1 | 26 | 85,8 | 520 |
| | $ICH_3$=1g | 2 | 33,5 | 92,5 | 325 |
| | FOMe=500 mmoles | 3 | 36,8 | 92,7 | 245 |
| | NMP=20cm$^3$ | | | | |
| | T=180°C | | | | |
| | PCO=1,5 MPa | | | | |
| 4 | $RhCl_3.3H_2O$= | | | | |
| | 0,125 mmole | 0,5 | 15,6 | 79,5 | 414 |
| | LiI=1,5g | 1 | 34,8 | 81,6 | 462 |
| | NMP=20cm$^3$ | 2,5 | 39,1 | 84,6 | 207 |
| | FOMe=166 mmoles | | | | |
| | T=180°C | | | | |
| | PCO=5 MPa | | | | |

| | | | | | |
|---|---|---|---|---|---|
| 5 | $RhCl_3.3H_2O$= | | | | |
| | 0,15 mmole | 1,5 | 34 | 93,3 | 373 |
| | LiI=2g | 2,5 | 71,1 | 80,4 | 468 |
| | FOMe=245 mmoles | 3,5 | 92 | 50,1 | 432 |
| | NMP=15cm$^3$ | | | | |
| | T=196°C | | | | |
| | PCO=10 MPa | | | | |
| 6 | $RhCl_3.3H_2O$= | | | | |
| | 0,15 mmole | 3,5 | 34,5 | 81,7 | 162 |
| | LiI=1g | 4,5 | 52 | 80,3 | 190 |
| | FOMe=247 mmoles | 5,5 | 72,6 | 71,9 | 217 |
| | NMP=15cm$^3$ | | | | |
| | T=196°C | | | | |
| | PCO=30 MPa | | | | |
| 7 | $RhCl_3.3H_2O$= | | | | |
| | 0,15 mmole | 3 | 71,7 | 92,5 | 393 |
| | LiI=0,25g | 4 | 81,9 | 90,6 | 337 |
| | FOMe=247 mmoles | 5 | 88,5 | 81,4 | 291 |
| | NMP=15cm$^3$ | | | | |
| | T=196°C | | | | |
| | PCO=30 MPa | | | | |

FOMe = formiate de méthyle

NMP = N-méthylpyrrolidone

Exemple 8 :

On a opéré comme à l'exemple 1 mais en utilisant 0,065 mmole de catalyseur $RhCl_3$, $3H_2O$, 180 mmole de formiate de méthyle, 50 cm$^3$ de solvant NMP, à une température de 184°C, sous une pression de CO de 5 MPa. On a fait varier la quantité de promoteur iodé LiI et on a déterminé le pourcentage de conversion en acétate de méthyle et la sélectivité au bout de 2h.

| Rapport promoteur catalyseur | % Conversion en acétate de méthyle | Sélectivité en acétate de méthyle % |
|---|---|---|
| 40 | 5 | 14 |
| 100 | 30 | 70 |
| 200 | 30 | 84 |

Exemple 9 :

A titre de comparaison, on a opéré comme à l'exemple 1 mais en utilisant 1,3 mmoles de $RhCl_3$, $3H_2O$; 820 mmoles de formiate de méthyle sans solvant à 180°C et 20 MPa de CO. On n'obtient pratiquement que de l'acide acétique, ce qui montre bien l'influence du solvant.

| Conditions expérimentales | t (h) | Conversion totale % | Sélectivité molaire % en acétate de méthyle | Sélectivité molaire % acide acétique |
|---|---|---|---|---|
| $RhCl_3.3H_2O$= 1,30 mmoles | 1 | 72 | 2,2 | 97,9 |
| LiI=2,8g FCMe=820 mmoles | 2 | 96,5 | 0,5 | 99,5 |
| T=180°C P=20 MPa | 3 | 99,3 | 0,3 | 99,7 |

**Revendications**

1. Procédé de préparation d'acétate de méthyle à partir de formiate de méthyle, caractérisé en ce que l'on convertit le formiate de méthyle à une température de 170 à 220°C en présence de :

a) un catalyseur à base de rhodium,

b) un promoteur iodé choisi parmi les iodures alcalins, alcalino-terreux, de phosphonium ou d'ammonium et les composés covalents d'iode additionnés d'une phosphine ou d'une amine tertiaire, ce promoteur étant présent à une concentration molaire de 0,05 à 1 mole/litre.

c) un solvant choisi parmi un N-alkyl amide cyclique.

2. Procédé selon la revendication 1, caractérisé en ce que le promoteur est un iodure alcalin ou alcalino-terreux.

3. Procédé selon le revendication 1, caractérisé en ce que le promoteur est un composé covalent d'iode en mélange avec une amine tertiaire.

4. Procédé selon la revendication 1, caractérisé en ce que le promoteur est un composé colvalent d'iode en mélange avec une phosphine.

5. Procédé selon la revendications 1 ou la revendication 2, caractérisé en ce que le solvant est utilisé en une portion molaire par rapport au formiate de méthyle de 10 à 50%.

6. Procédé selon l'une quelconque des revendications 1 à 5, caractérisé en ce que l'on opère sous une pression de CO de $1.10^5$ à $20.10^5$ Pa.

## Patentansprüche

1. Verfahren zur Herstellung von Methylacetat, ausgehend von Methylformiat, dadurch gekennzeichnet, daß man das Methylformiat bei einer Temperatur von 170 bis 220°C in Gegenwart

a) eines Katalysators auf Rhodiumbasis,

b) eines jodhaltigen Promotors ausgewählt aus den Alkali-, Erdalkali-, Phosphonium- oder Ammoniumjodiden und den kovalenten Jodverbindungen unter beimischung eines Phosphins oder eines tertiären Amins, wobei der Promotor in einer molaren Konzentration von 0,05 bis 1 Mol/Liter zugegen ist,

c) eines Lösungsmittels ausgewählt aus einem zyklischen N-Alkylamid umwandelt.

2. Verfahren nach Anspruch 1, dadurch gekennzeichnet, daß der Promotor ein Alkali- oder Erdalkalijodid ist.

3. Verfahren nach Anspruch 1, dadurch gekennzeichnet, daß der Promotor eine kovalente Jodverbindung im Gemisch mit einem tertiären Amin ist.

4. Verfahren nach Anspruch 1, dadurch gekennzeichnet, daß der Promotor eine kovalente Jodverbindung im Gemisch mit einem Phosphin ist.

5. Verfahren nach Anspruch 1 oder Anspruch 2, dadurch gekennzeichnet, daß das Lösungsmittel in einem molaren Anteil bezogen auf das Methylformiat von 10 bis 50 % verwendet wird.

6. Verfahren nach irgendeinem der Ansprüche 1 bis 5, dadurch gekennzeichnet, daß man unter einem Druck von CO von $1.10^5$ bis $20.10^5$ Pa arbeitet.

## Claims

1. Process for preparing methyl acetate from methyl formate, characterised in that methyl formate is converted at a temperature of 170 to 220°C in the presence of:

a) a rhodium-based catalyst,

b) an iodine-containing promoter chosen from alkali metal, alkaline earth metal, phosphonium or ammonium iodides and covalent iodine compounds to which a phosphine or a tertiary amine is added, this promoter being present at a molar concentration of 0.05 to 1 mol/litre,

c) a solvent chosen from a cyclic N-alkylamide.

2. Process according to Claim 1, characterised in that the promoter is an alkali metal iodide or alkaline earth metal iodide.

3. Process according to Claim 1, characterised in that the promoter is a covalent iodine compound mixed with a tertiary amine.

4. Process according to Claim 1, characterised in that the promoter is a covalent iodine compound mixed with a phosphine.

5. Process according to Claim 1 or Claim 2, characterised in that the solvent is used in a molar proportion relative to the methyl formate of 10 to 50%.

6. Process according to any one of Claims 1 to 5, characterised in that the reaction is performed under a CO pressure of $1.10^5$ to $20.10^5$ Pa.